# NEW EUROPEAN PATENT SPECIFICATION

(11) **EP 1 153 930 B2**
(45) Date of publication and mention of the opposition decision: **28.03.2007**
(45) Mention of the grant of the patent: 23.04.2003
(21) Application number: 01304284.1
(22) Date of filing: 14.05.2001
(51) Int. Cl.: C07H 3/00, C12P 19/16, C12P 19/02

(54) **Method for enzymatically hydrolysing mixtures of isomaltulose and trehalulose**
Verfahren zur enzymatischen Hydrolyse von Mischungen aus Isomaltulose und Trehalulose
Procédé pour l'hydrolyse enzymatique de mélanges de isomaltulose et tréhalulose

(30) Priority: 13.05.2000 GB 0011468
(43) Date of publication of application: 14.11.2001
(73) Proprietor: Cerestar Holding B.V., 4551 LA Sas Van Gent (NL)
(72) Inventor: Vercauteren, Ronny Leontina Marcel, 9120 Beveren (BE); NGuyen, Van Sau, 1070 Brussels (BE); Heylen, An Amanda Jules, 1800 Vilvoorde (BE)
(74) Representative: Wilkinson, Stephen John

(56) References cited:
- EP-A- 0 109 009
- EP-A- 0 794 259
- YAMADA K ET AL: "HYDROLYSIS OF 1-O-ALPHA-D GLUCOPYRANOSYL-D-FRUCTOFURANOSE TREHALULOSE BY RAT INTESTINAL SUCRASE-ISOMALTASE COMPLEX" NUTRITION REPORTS INTERNATIONAL, vol. 32, no. 5, 1985, pages 1211-1220, XP000971129 ISSN: 0029-6635
- SIEBERT G ET AL: "TIERED SCREENING OF SUGAR SUBSTITUTES PRESCREENING WITH ENZYMES 1. ALPHA GLUCOSIDASE FROM YEAST" ZEITSCHRIFT FUER ERNAEHRUNGSWISSENSCHAFT, vol. 25, no. 4, 1986, pages 242-247, XP000971108 ISSN: 0044-264X
- WEIDENHAGEEN, LORENZ: "Palatinose (6-(a-glucopyranosido)-fructofuranose), ein neues bakterielles Umwandlungsprodukt der Saccharose" ZUCKERINDUSTRIE, vol. 82, no. 11, 20 November 1957 (1957-11-20), pages 533-534, XP000971144
- DAHLQVIST: "Hydrolysis of palatinose (isomaltulose) by pig intestinal glycosidases" ACTA CHEMICA SCANDINAVICA, vol. 15, no. 6, pages 808-808-816, XP000971325
- GODA T ET AL: "HYDROLYSIS OF ALPHA-D GLUCOPYRANOSYL-1 6-SORBITOL AND ALPHA-D GLUCOPYRANOSYL-1 6-MANNITOL BY RAT INTESTINAL DISACCHARIDASES" JOURNAL OF NUTRITIONAL SCIENCE AND VITAMINOLOGY, vol. 34, no. 1, 1988, pages 131-140, XP000971173 ISSN: 0301-4800

## Description

### Technical field

The present invention relates to a method for converting enzymatically a mixture comprising isomaltulose and trehalulose into a syrup containing at least 10% w/w of glucose and fructose. The enzyme or combination of enzymes is applied in batch or in immobilised form.

### Background of the invention

Isomaltulose or 6-O-α-D-glucopyranosyl-D-fructofuranose is synthesised from sucrose by the action of an enzyme present in bacterial strains like *Protaminobacter* rubrum, *Erwinia* rhapontici and *Serratia* plymuthica.

The sucrose glucosylmutase (also called isomaltulose synthase, sucrose mutase or sucrose isomerase (E. C. 5.4.99.11)) present in these microorganisms is responsible for the rearrangement of the α1→β2 glycosidic linkage in sucrose, to an α1→6 linkage in isomatulose. Isomaltulose is the kinetically formed product. However, also a quantity of the thermodynamically more stable product trehalulose is formed.

Trehalulose is 1-a-glucopyranosyl-fructose and contains an α1 → 1 linkage between the glucopyranosyl and the fructopyranose moieties.

Mixtures comprising glucosylfructoses, such as isomaltulose and trehalulose are obtained for example during the production process for isomalt (hydrogenated isomaltulose), i.e. the quasi-equimolar mixture of 6-O-α-D-glucopyranosyl-D-sorbitol (1,6 GPS), and 1-O-α-D-glucopyranosyl-D-mannitol (1,1 GPM). Isomalt is currently used as a low-calorie sweetener and is known for its non-cariogenicity and enhanced stability as compared to sucrose.

EP 0 625 578 describes a method for preparing hydrogenated isomaltulose. In a first method step saccharose (i.e. sucrose) is enzymatically converted into a saccharide mixture, which is termed "isomerised saccharose" and contains trehalulose and isomaltulose. In a second step the "isomerised saccharose" is freed from non-isomerised remaining saccharose by enzymatic and/or H⁺-catalysed hydrolysis. In a further step the "isomerised saccharose" is catalytically hydrogenated, in which case preferably either before or after the catalytic hydrogenation, the mixture, which is obtained, is subjected to chromatographic separation.

H. Schiweck describes in Alimenta 19, (1980) p 5-16 a purification of the "isomerised saccharose" containing syrup by the crystallisation of the isomaltulose fraction from supersaturated aqueous solutions. The mother liquor still contains isomaltulose, isomaltose, trehalulose, glucose, fructose, and a little quantity of residual saccharose. The presence in the mother liquor of residual isomaltulose and trehalulose, both being disaccharides which are difficult to hydrolyse and difficult to ferment, makes it rather difficult to use it as an alternative fermentation substrate, for the production of foods or pharmaceuticals like antibiotics.

A route to circumvent this problem is to convert the remaining isomaltulose and trehalulose into a syrup containing the fermentable monosaccharides glucose and fructose, and apply the thus obtained syrup directly for applications in food, feed and fermentation.

H⁺-catalysis or enzymatic hydrolysis of glycosidic bonds is facilitated when there is a great energy-release during the hydrolysis of the corresponding bond. However, such an energy-release is almost negligible for α-1-6- and α-1-1-bonds, consequently the enzymatic hydrolysis of isomaltulose and trehalulose will be very difficult.

R. Weidenhageen describes in Zeitschrift für die Zuckerindustrie, LXXXII, (1957), p 533 that isomaltulose can be enzymatically hydrolysed with alpha-glucosidase from brewer's yeast, but with lower velocity than the hydrolysis of maltose. Nothing is reported on the hydrolysis of trehalulose.

S. Sugawara describes in Journal Facul. Agr. Hokkaido, vol. 52 part 3, (1962), p 257-321 studies on the mode of occurrence of alpha-glucosidase activities in the fungus *Aspergillus* oryzae. He reports the very slow hydrolysis of isomaltulose by an alpha-glucosidase from *Aspergillus* oryzae.

G. Siebert et. al. describes in Zeitschrift fur Ernährungswissenschaft **25**, (1986), p242-247 the very slow hydrolysis of isomaltulose and trehalulose by α-glucosidase from yeast. In a similar reaction set-up where maltose is hydrolysed to an extent of 100% trehalulose is hydrolysed to an extent of 6% and isomaltulose is hydrolysed to an extent of only 3%.

This method thus applied has in fact several disadvantages. The hydrolysis of α-1-6 or α-1-1 glycosidic bonds such as in isomaltulose and trehalulose is very low, 3% and 6%, respectively whereas the hydrolysis has to be conducted at a temperature- and pH-optimum where microbial growth is stimulated, resulting in an increased risk of microbial contamination of the hydrolysed syrup.

Consequently, there is a clear need for an industrially viable method consisting of a simple enzymatic hydrolysis of hydrolysing 1-6 and α-1-1 glycosidic bonds into a valuable glucose fructose containing syrup, which can directly be applied in food, feed and fermentation.

The current invention provides such a method.

### Summary of the invention

The present invention discloses a method for enzymatically hydrolysing a substrate wherein said method comprises the following steps:
a) Taking a substrate comprising a mixture of isomaltulose and trehalulose comprising at least 20% w/w isomaltulose and trehalulose,
b) Bringing said substrate into contact with an isomaltulose and trehalulose hydrolysing to hydrolyse alpha-glucosidase from eubacteria archaeobacteria or fungi,
c) Maintaining said contact to hydrolyse the substrate to obtain syrup containing more than 10% w/w, preferably more than 25% w/w, more preferably more than 40% w/w, even more preferably more than 60% w/w, most preferably more than 80% w/w glucose and fructose, and
d) Optionally, concentrating said syrup.

The present invention relates to a method wherein the syrup containing fructose and glucose is chromatographically purified into a fraction containing fructose and glucose, and a by-stream comprising isomaltulose and trehalulose, and said by-stream is recycled into step b).

The present invention further relates to a method whereby method of isomaltulose and thehalulose comprising between 15% to 70% w/w isomaltulose and 5% to 30% w/w trehalulose are hydrolysed.

Furthermore, the current invention discloses a method for enzymatically hydrolysing a mixture; which consists of the dry substance of the mother liquor obtained from the crystallisation of isomaltulose.

The obtained glucose fructose containing syrup comprises less than 30% w/w residual isomaltulose and trehalulose, preferably less than 20% w/w isomaltulose and trehalulose, more preferably less than 10% w/w isomaltulose and trehalulose.

The current invention relates to a method characterised In that the substrate is brought into contact with an isomaltulose and trehalulose hydrolysing alpha-glucosidase and is converted at pH below 6.5, preferably below 6, more preferably below 5 and at a temperature higher than 30°C, preferably higher than 40°C, more preferably at a temperature higher than 50°C into a syrup containing fructose and glucose.

The method of the current invention is further characterised in that the alpha-glucosidase is obtained from fungi preferably from the genera Aspergillus, Penicillum, Candida, Monilia, Phoma and Alternaria, more preferably from the species *Aspergillus niger, Aspergillus oryzae* or *Aspergillus awamori.*

The current invention further relates to a method wherein the alpha-glucosidase is obtained from eubacteria, preferably from the genera Enterobacter, Escherichia, Actinomyces, Bacillus, Klebsiella, and Arthrobacter, more preferably from the species *Bacillus subtilis, Bacillus licheniformis, and Bacillus thermoamyloliquefaciens.*

The method of the current invention is further characterised in that the alpha-glucosidase is obtained from archaeobacteria, preferably from genera Thermococaus, Thermoproteus, Pyrodictium and Halobacteria.

The current invention further relates to a method wherein the isomaltulose and trehalulose enzyme is used In immobilised form, preferably immobilised on a carrier, more preferably immobilised on a re-usable carrier.

The present invention further relates to a method wherein an alpha-glucosidase is immobilised on anion exchange resin.

The current invention discloses a method wherein an alpha-glucosidase is immobilised on an anion exchange resin and the syrup containing glucose and fructose contains more than 80% w/w glucose and fructose and less than 20% w/w, preferably less than 10% w/w isomaltulose and trehalulose and, these values are achieved with a flow-rate of up to 5 bed-volumes per hour and for a period of at least 60 days.

According to the current invention the obtained glucose fructose syrup may be further refined i.e. treated by chromatographic means or by filtration.

The current invention further relates to the use of an isomaltulose and trehalulose hydrolysing having these activities for hydrolysing a substrate comprising a mixture of isomaltulose and trehalulose alpha-glucosidase from eubacteria, archaeobacteria or fungi comprising at least 20% w/w isomaltulose and trehalulose into a syrup containing more than 10% w/w, preferably more than 25% w/w, more preferably more than 40%, even more preferably more than 60% w/w, most preferably more than 80% w/w glucose and fructose.

### Detailed description of the invention

The present invention discloses a method for enzymatically hydrolysing a substrate characterised in that said method comprises the following steps:
a) Taking a substrate comprising a mixture of isomaltulose and trehalulose comprising at least 20% w/w isomaltulose and trehalulose,
b) Bringing said substrate into contact with an isomaltulose and trehalulose hydrolysing alpha-glucosidase from eubacteria, archaeobacteria or fungi,
c) Maintaining said contact to hydrolyse the substrate to obtain a syrup containing more than 10% w/w, preferably more than 25% w/w more preferably more than 40% w/w even more preferably more than 60% w/w, most preferably or more than 80% w/w glucose and fructose, and
d) Optionally, concentrating said syrup.

The enzymatic hydrolysis is affected by a specific isomaltulose and trehalulose hydrolysing enzyme. The isomaltulose and trehalulose hydrolysing enzyme can be any enzyme capable of hydrolysing the alpha 1-1 and the alpha 1-6 glycosidic bonds, and its use is leading to an industrial process.

The syrup containing glucose and fructose is concentrated so that the microbial contamination is avoided. Particularly the syrup is concentrated to a dry substance more than 20% w/w, preferably to a dry substance of more than 30% w/w, more preferably more than 40% w/w.

The present invention is further characterised in that between step c) and step d) or after step d) the syrup is chromatographically purified into a fraction containing fructose and glucose, and a by-stream comprising isomaltulose and trehalulose, and said by-stream can be recycled into step b).

The present invention further relates to a method whereby mixtures of isomaltulose and trehalulose comprising, or between 15% to 70% w/w isomaltulose and 5% to 30% w/w trehalulose are hydrolysed.

These mixtures may be the dry substance of the residual mother liquor obtained by the crystallisation of the isomaltulose fraction from supersaturated aqueous solutions of the "isomerised saccharose" containing syrup. Such a mother liquor still can contain between 15% and 70% w/w isomaltulose, between 0-10% w/w isomaltose, between between 5% and 30% w/w trehalulose, between 2% and 15% w/w glucose, between 2% and 15% w/w fructose, and between 0% and 5% w/w residual saccharose, while other oligosaccharides might be present as well. In a typical example, the dry substance of such mother liquor comprises 53% w/w isomaltulose and 17.5% w/w trehalulose. These relatively high quantities of isomaltulose and trehalulose present in the mother liquor makes it difficult to use it as an alternative fermentation substrate, for the production of foods or pharmaceuticals like antibiotics.

The current invention provides an enzymatic method for converting this by-product stream, without direct value into a valuable glucose fructose syrup which can directly be applied into feed, food and fermentation.

The current invention demonstrates that the isomaltulose and trehalulose hydrolysing enzyme is operational at favourable temperature and pH reaction conditions and where microbial contamination is avoided.

The current invention relates to a method characterised in that the substrate is brought into contact with an isomaltulose and trehalulose hydrolysing enzyme having these activities and is converted into a syrup containing fructose and glucose at pH below 6.5, preferably below 6, more preferably below 5 and at a temperature higher than 30°C, preferably higher than 40°C, more preferably higher than 50°C.

These alpha-glucosidases (transglucosidases) can be applied at a temperature and pH optimum where microbial contamination is quasi excluded. For example alpha-glucosidase from *Aspergillus* oryzae has a pH optimum around pH 42-4.6, and a temperature optimum of about 55°C. However, up to now the applied reaction conditions for the hydrolysis by these enzymes from bacterial or fungal origin results in a far too slow hydrolysis for obtaining an industrial viable process. And the described reactions applied only pure substrates.

Alpha-glucosidase obtained from fungi, preferably from genera Aspergillus, Penicillum Candida, Monilia, Phoma, and Alternaria, more preferably from the species *Aspergillus niger, Aspergillus oryzae* or *Aspergillus awamori* can be applied in an industrial viable process. Furthermore, alpha-glucosidase obtained from eubacteria, preferably from the genera Enterobacter, Escherichia, Actinomyces, Bacillus, Klebsiella, and Arthrobacter, more preferably from the species *Bacillus subtilis, Bacillus licheniformis,* and *Bacillus thermoamyloliquefaciens* can be applied as well.

The method of the current invention is further characterised in that the alpha-glucosidase is obtained from archaeobacteria, preferably from the genera Thermococcus, Thermoproteus, Pyrodictium and Halobacteria.

The enzymatic hydrolysis of mixtures comprising isomaltulose and trehalulose results in syrups containing more than 10% w/w, preferably more than 25% w/w, more preferably more than 40% w/w glucose and fructose. The method of the current invention applies an enzyme, which needs no further purification and which can be applied as commercial base, and whereby mixtures of isomaltulose and trehalulose are applied at high dry substance (i.e. at least 20% w/w). The hydrolysis is resulting in syrups containing even more preferably more than 60% w/w, most preferably more than 80% /w glucose and fructose, i.e. a significant degree of hydrolysis is obtained.

Furthermore the syrup containing fructose and glucose comprises less than 30% w/w residual isomaltulose and trehalulose, preferably less than 20% w/w isomaltulose and trehalulose, more preferably less than 10% w/w isomaltulose and trehalulose.

The industrial viability of the current invention is further improved by applying isomaltulose and trehalulose hydrolysing enzyme, in immobilised form. The isomaltulose and trehalulose hydrolysing enzyme may be available in immobilised form such as cross-linked enzyme crystals. The enzyme can be immobilised on a carrier, more preferably immobilised on a re-usable carrier. For the present purpose re-usable means that the carrier can be freed of enzyme or enzymatic activity in such a way that the carrier material stays intact. The carrier may be either continuously or intermittently refreshed. The carrier material can then be re-loaded with enzyme and re-used. The carrier material can be re-loaded with enzyme and re-used. The cleaning of the carrier can for example be performed by washing with acidic or basic solution. This may be done in batch or in the column. It may be advantageous to add a salt. Another possibility is the use of protein degrading enzymes. Yet, another means would be heating of the material.

Preferably, the carrier is inert in the sense that it should not affect the conversion of the substrate into the syrup containing fructose and glucose.

Furthermore, the carrier is preferably described as being porous and substantially non-compressible. The term 'porous' is intended to mean that the solid carrier comprises a multitude of hollows and pores providing a large surface area. An example of the use of a porous material is the magnetically stabilised fluidised bed enzyme reactor. The term substantially non-compressible is intended to mean that the solid carrier does not deform to any appreciable extent at the pressure, which might prevail during the conversion process.

Preferably, materials are used which have anion exchange groups. Such materials may be on the basis of cellulose. Other more preferred carriers are polyacrylate or polystyrene based carriers having weakly basic groups. Weakly basic anion exchangers are materials having primary and/or secondary or/tertiary amino groups. They dissociate and have exchange capability in acidic solutions. The materials having tertiary amino groups have rather basic properties and they are also called medium basic anion exchangers. Preferably, phenolformaldehyde based carriers are used, such as commercial available Duolite^{™} A 568 (Rohm and Haas).

Also, a treatment with a cross-linking agent, such as glutaric dialdehyde can be performed to stabilise the immobilised enzymes.

The method comprising an immobilised alpha-glucosidase is demonstrated by example 2 of the current invention. The enzyme is immobilised onto an anion exchange resin, e.g. macroporous weak anion exchanger.

When passing the substrate comprising a mixture of isomaltulose and trehalulose through a column filled with immobilised alpha-glucosidase conjugate, the fructose glucose syrup contains more than 80% w/w glucose and fructose and less than 30% w/w residual isomaltulose and trehalulose, preferably less than 20% w/w isomaltulose and trehalulose, more preferably less than 10% isomaltulose and trehalulose. These values are achieved with a flow-rate up to 5 bed volumes per hour, preferably up to 2 bed volume per hour and for a period of at least 60 days.

When passing a substrate-solution of 20% (w/w) at a pH of 4.5 through a column filled with immobilised alpha-glucosidase conjugate (immobilised on an anion exchange resin such as Duolite ^{™} A 568), the enzyme system remains stable for at least 62 days by applying a flow-rate of 0.3 bed volumes per hour and at least 85% w/w fructose and glucose is obtained.

When applying isomaltulose and trehalulose hydrolysing enzyme in immobilised form, such as cross-linked enzyme crystals, the syrup containing fructose and glucose can be obtained by applying flow-rates even higher than 5 bed-volumes per hour.

The present invention further relates to a method whereby the mixture of isomaltulose and trehalulose comprises at least 30% w/w isomaltulose and trehalulose.

The method is further characterised in that the mixture comprises between 15% to 70% w/w isomaltulose and 5% to 30% w/w trehalulose and the degree of hydrolysis is sufficiently high for obtaining a glucose fructose containing syrup, which comprises less than 30% isomaltulose and trehalulose, preferably less than 20% w/w residual isomaltulose and trehalulose, more preferably less than 10% w/w isomaltulose and trehalulose.

According to the current invention the obtained glucose fructose syrup may be further refined i.e. treated by chromatographic means or by filtration for obtaining a syrup with high content of fructose and glucose. A chromatographic separation of hydrolysed isomaltulose mother liquor is presented in example 3. Simulated moving bed chromatography using a strong acidic ion-exchange resin in the Na⁺-form is applied and a syrup containing about 57% fructose and 42.8% glucose is obtained, i.e. 99.8% monosaccharide purity. The resulting by-product stream, the raffinate, which is comprising fructose and glucose, isomaltulose, trehalulose, isomaltose and residual higher molecular weight oligosaccharides, is submitted to a second hydrolysing step by alpha-glucosidase and thus the overall recovery of glucose and fructose is further increased.

The composition of the mixture comprising isomaltulose and trehalulose and the glucose fructose syrup has been determined by applying HPLC (Dionex Ion chromatograph equipped with amperometric detector and Carbopac column).

The current invention further relates to the use of an isomaltulose and trahalulose hydrolysing enzyme or a combination of enzymes having these activities for hydrolysing a substrate comprising a mixture of isomaltulose and trehalulose comprising at least 20% w/w isomaltulose and trehalulose into a syrup substrate comprising a containing more than 10% w/w, preferably more than 25% w/w, more preferably more than 40% w/w, even more preferably more than 60% w/w glucose and fructose, most preferably more than 80% w/w glucose and fructose.

The current invention results in an industrial viable process with significant advantages:
- By hydrolysing the dry substance of the mother liquor obtained from the crystallisation of isomaltulose, the current invention is adding-up to the industrial viability of the industrial production process of isomalt. The current invention converts a by-product stream of no value, into a glucose fructose syrup which can directly be applied in food, feed or fermentation.
- The hydrolysis is performed on mixtures of isomaltulose and trehalulose.
- The mixtures are applied at high dry substance, i.e at least 20% w/w.
- The reaction conditions in respect of pH- and temperature-optimum are such that the hydrolysis is devoid of microbial contamination.
- The degree of hydrolysis is sufficiently high to obtain syrups containing more than 10% w/w, 25% w/w, or 40% w/ w glucose and fructose.
- The degree of hydrolysis in presence of immobilised enzyme remains constant for at least 60 days, and is resulting in a syrup with a constant composition.
- Industrial viable process

The current invention is demonstrated by the following examples.

### Example 1- Dosage of alpha-glucosidase

Batch incubations were performed with the alpha-glucosidase enzyme from Amano (Transglucosidase L "DAmano" - EC 3.2.1.20 - 300,000 Amano units permi of product). The applied substrate had the following composition:

| **Fructose % w/w** | **Glucose % w/w** | **Isomaltulose + trehalulose % w/w** |
|---|---|---|
| 10 | 6.7 | 76.7 |

The hydrolysis experiments were conducted at 30% d.s., pH 5 and 60°C for 24, 48 and 72 hours. The obtained results are displayed in Table 1.

**Table 1**

| **Dosage enzyme on dry substance (% w/w)** | **Temp °C.** | **Time (h)** | **Fructose** | **Glucose** | **DP2** | **% Degree Hydrolysis** |
|---|---|---|---|---|---|---|
| | - | - | 10 | 6.7 | 76.7 | 0 |
| 0.2 | 60 | 24 | 14.8 | 10.7 | 60.6 | 11.5 |
| | | 48 | 17 | 12.8 | 55.3 | 17.1 |
| | | 72 | 18.5 | 12.3 | 53 | 18.4 |
| | | | | | | |
| 0.5 | 60 | 24 | 18.4 | 12.9 | 53 | 19.0 |
| | | 48 | 21.6 | 16 | 46.9 | 27.2 |
| | | 72 | 23.8 | 17.3 | 44 | 31.8 |
| | | | | | | |
| 1.0 | 60 | 24 | 22.1 | 15.7 | 46 | 27.5 |
| | | 48 | 26.3 | 20 | 39.6 | 38.6 |
| | | 72 | 28.8 | 22.3 | 36.2 | 44.9 |
| | | | | | | |
| 1.7 | 60 | 24 | 26.4 | 19.7 | 39.6 | 38.3 |
| | | 48 | 31.4 | 25.2 | 31.5 | 52.0 |
| | | 72 | 33.9 | 28 | 28.6 | 58.9 |

### Example 2 - Hydrolysis with immobilised alpha-glucosidase

### 1. Immobilisation.

The Transglucosidase-L Amano (0.6 g of Transglucosidase solution for 1 ml of enzyme carrier) was immobilised on Duolite^{™} A 568 (a macroporous weak anion exchanger). The conjugate was stirred for 6 hours and 0.08 ml glutaric aldehyde (25%)/g conjugate was added. Stirring was continued for 24 hours. The thus obtained conjugate was washed with 4 bed volumes of demineralised water.

### 2. Hydrolysis

Substrate with the following composition was used:

| **Fructose % w/w** | **Glucose % w/w** | I**somaltulose + trehalulose % w/w** |
|---|---|---|
| 10 | 6.7 | 76.7 |

The substrate solution of 20% (w/w) at a pH of 4.5 was passed through a column filled with immobilised Transglucosidase conjugate. The temperature of the column was maintained at 50°C and the flow-rate was kept constant at 0.3 bed-volumes per hour.
The obtained results are shown in the table 2

**Table 2**

| **DAYS** | **Fructose %w/w** | **Glucose % w/w** | **Isomaltulose + trehalulose % w/w** |
|---|---|---|---|
| 1 | 44.7 | 42.7 | 5.3 |
| 2 | 44.7 | 43.2 | 4.8 |
| 13 | 44.3 | 43.0 | 5.1 |
| 14 | 44.8 | 43.6 | 5.5 |
| 15 | 44.3 | 43.4 | 5.6 |
| 16 | 43.9 | 43.0 | 6.3 |
| 19 | 44.0 | 41.9 | 6.8 |
| 22 | 43.9 | 42.1 | 6.1 |
| 23 | 45.2 | 44.3 | 4.2 |
| 27 | 44.2 | 42.5 | 6.1 |
| 29 | 44.2 | 42.5 | 6.5 |
| 30 | 44.4 | 43.0 | 6.0 |
| 34 | 44.3 | 42.4 | 6.9 |
| 35 | 44.3 | 42.4 | 5.6 |
| 36 | 44.0 | 42.4 | 6.0 |
| 37 | 44.2 | 42.5 | 6.0 |
| 40 | 43.9 | 41.9 | 6.7 |
| 41 | 44.1 | 42.4 | 6.0 |
| 42 | 43.9 | 42.2 | 6.3 |
| 43 | 43.0 | 41.4 | 7.1 |
| 44 | 43.5 | 41.9 | 7.3 |
| 47 | 43.4 | 41.5 | 7.2 |
| 48 | 44.1 | 43.0 | 4.8 |
| 49 | 44.2 | 42.5 | 6.0 |
| 50 | 43.8 | 42.2 | 6.3 |
| 53 | 43.6 | 42.0 | 6.8 |
| 54 | 43.7 | 41.8 | 7.0 |
| 56 | 44.3 | 41.7 | 6.8 |
| 57 | 44.1 | 42.0 | 6.7 |
| 60 | 45.5 | 42.3 | 6.2 |
| 61 | 44.8 | 42.0 | 6.4 |
| 62 | 44.5 | 42.1 | 6.5 |

### Example 3 - Chromatographic separation.

A glucose fructose syrup with the following composition was applied for chromatographic separation:

| Fructose (% w/w) | Glucose (% w/w) | Isomaltulose + trehalulose(% w/w) |
|---|---|---|
| | | |
| 41.8 | 36.7 | 14.6 |

This syrup was fractionated, by simulated moving bed chromatography, using a strong acidic ion-exchanger in the Na⁺- form, into a product fraction and a by-stream (raffinate). The obtained product fraction had the following composition: 99.8% monosaccharides (w/w), of which was 57 % w/w fructose and 42.8 % w/w glucose.
The residual by-stream (raffinate) had the following composition:

| Fructose + Glucose (% w/w) | Isomaltulose + trehalulose + isomaltose (% w/w) | ≥ DP3+ (% w/w) |
|---|---|---|
| 29.4 | 58.8 | 11.8 |

The raffinate was passed through an immobilised Transglucosidase conjugate at 40°C and at 30% d.s. (see example 2 for a description of the Transglucosidase conjugate) and was converted into a high fructose/glucose syrup with the following composition:

| Flow rate (BV/h) | Fructose + Glucose (% w/w) | Isomaltulose + trehalulose + isomaltose (% w/w) | ≥ DP3+ (% w/ w) |
|---|---|---|---|
| 0.1 | 74.5 | 18.5 | 7.0 |
| 0.3 | 64.2 | 25.1 | 8.3 |

## Claims

1. A method for enzymatically hydrolysing a substrate, **characterised in that** said method comprises the following steps:
a) taking a substrate comprising a mixture of isomaltulose and trehalulose comprising at least 20% w/w isomaltulose and trehalulose;
b) bringing said substrate into contact with an isomaltulose- and trehalulose- hydrolysing alpha-glucosidase from eubacteria, archaeobacteria or fungi;
c) maintaining said contact to hydrolyse the substrate to obtain a syrup containing more than 10% w/w. preferably more than 25% w/w, more preferably more than 40% w/w, even more preferably more than 60% w/w, most preferably more than 80% w/w glucose and fructose;
d) optionally, concentrating said syrup.

2. A method according to claim 1, **characterised in that** between step c) and step d) or after step d) the syrup is chromatographically purified into a fraction containing fructose and glucose, and a by-stream comprising isomaltulose and trehalulose, and said by-stream is recycled into step b).

3. A method according to either claim 1 or claim 2, **characterised in that** the mixture comprises between 15% to 70% w/w isomaltulose and 5% to 30% w/w trehalulose.

4. A method according to any one of claims 1 to 3, **characterised in that** the mixture is the dry substance of the mother liquor obtained from the crystallisation of isomaltulose.

5. A method according to any one of claims 1 to 4 **characterised in that** the syrup containing glucose and fructose comprises less than 30% w/w isomaltulose and trehalulose, preferably less than 20% w/w isomaltulose and trehalulose, more preferably less than 10% w/w isomaltulose and trehalulose.

6. A method according to any one of claims 1 to 5, **characterised in that** the substrate is brought into contact with the isomaltulose- and trehalulose- hydrolysing alpha-glucosidase and contact is maintained at a pH below 6.5, preferably below 6, more preferably below 5 and at a temperature higher than 30°C, preferably higher than 40°C, more preferably higher than 50°C to obtain a syrup containing fructose and glucose.

7. A method according to claim 1, **characterised in that** the isomaltulose- and trehalulose- hydrolysing alpha-glucosidase is obtained from fungi, preferably from the genera Aspergillus, Penicillum, Candida, Monilia, Phoma, and Alternaria, more preferably from the species *Aspergillus niger, Aspergillus oryzae* or *Aspergillus awamori.*

8. A method according to claim 1, **characterised in that** the isomaltulose- and trehalulose- hydrolysing alpha-glucosidase is obtained from eubacteria, preferably from the genera Enterobacter, Escherichia, Actinomyces, Bacillus, Klebsiella, and Arthrobacter, more preferably from the species *Bacillus subtilis, Bacillus licheniformis,* and *Bacillus thermoamyloliquefaciens.*

9. A method according to claim 1, **characterised in that** the isomaltulose- and trehalulose- hydrolysing alpha-glucosidase is obtained from archaeobacteria, preferably from the genera Thermococcus, Thermoproteus, Pyrodictium and Halobacteria.

10. A method according to claim 1, **characterised in that** the isomaltulose- and trehalulose- hydrolysing alpha-glucosidase is used in immobilised form, preferably immobilised on a carrier, more preferably immobilised on a re-usable carrier.

11. A method according to claim 10, **characterised in that** the carrier is an anion exchange resin.

12. A method according to claim 11, **characterised in that** the syrup containing glucose and fructose contains more than 80% w/w glucose and fructose and less than 20% w/w isomaltulose and trehalulose, and that these values are achieved with a flow-rate of up to 5 bed volumes per hour and for a period of at least 60 days.

13. A method according to any one of claims 1 to 12, wherein the syrup containing glucose and fructose is further refined i.e. treated by chromatographic means or by filtration.

14. Use of an isomaltulose- and trehalulose- hydrolysing alpha-glucosidase from eubacteria, archaeobacteria or fungi for hydrolysing a substrate comprising a mixture of isomaltulose and trehalulose comprising at least 20% w/w isomaltulose and trehalulose into a syrup containing more than 10% w/w, preferably more than 25% w/w, more preferably more than 40% w/w, even more preferably more than 60% w/w*,* most preferably more than 80% w/w glucose and fructose.

## Patentansprüche

1. Verfahren zum enzymatischen Hydrolysieren eines Substrats, **dadurch gekennzeichnet, dass** das Verfahren die nachstehenden Schritte umfasst:
a) Nehmen eines Substrats, umfassend ein Gemisch von Isomaltulose und Trehalulose, umfassend mindestens 20 % Gew./Gew. Isomaltulose und Trehalulose,
b) Inkontaktbringen des Substrats mit einer Isomaltulose und Trehalulose hydrolysierenden α-Glucosidase aus Eubacteria, Archaeobacteria oder Pilz,
c) Kontakthalten zum Hydrolysieren des Substrats, um einen Sirup, enthaltend mehr als 10 % Gew./Gew., vorzugsweise mehr als 25 % Gew./Gew., bevorzugter mehr als 40 % Gew./ Gew., stärker bevorzugt mehr als 60 % Gew./Gew., am stärksten bevorzugt mehr als 80 % Gew./Gew. Glucose und Fructose, zu erhalten,
d) gegebenenfalls Aufkonzentrieren des Sirups.

2. Verfahren nach Anspruch 1, **dadurch gekennzeichnet, dass** zwischen Schritt c) und Schritt d) oder nach Schritt d) der Sirup chromatographisch in eine Fructose und Glucose enthaltende Fraktion und einen Isomaltulose und Trehalulose umfassenden Nebenstrom gereinigt wird und wobei der Nebenstrom in Schritt b) zurückgeführt wird.

3. Verfahren nach Anspruch 1 oder 2, **dadurch gekennzeichnet, dass** das Gemisch mindestens 15 bis 70 % Gew./Gew. Isomaltulose und 5 bis 30 % Gew./Gew. Trehalulose umfasst.

4. Verfahren nach einem von Anspruch 1 bis 3, **dadurch gekennzeichnet, dass** das Gemisch die Trockensubstanz der aus der Kristallisation von Isomaltulose erhaltenen Mutterlauge ist.

5. Verfahren nach einem von Anspruch 1 bis 4, **dadurch gekennzeichnet, dass** der Glucose und Fructose enthaltende Sirup weniger als 30 % Gew./Gew. Isomaltulose und Trehalulose, vorzugsweise weniger als 20 % Gew./Gew. Isomaltulose und Trehalulose, bevorzugter weniger als 10 % Gew./Gew. Isomaltulose und Trehalulose, umfasst.

6. Verfahren nach einem von Anspruch 1 bis 7, **dadurch gekennzeichnet, dass** das Substrat mit einer Isomaltulose und Trehalulose hydrolysierenden α-Glucosidase in Kontakt gebracht wird und dieser Kontakt erhalten wird bei pH unter 6,5, vorzugsweise unter 6, bevorzugter unter 5 und bei einer Temperatur höher als 30°C, vorzugsweise höher als 40°C, bevorzugter höher als 50°C, um einen in einen Fructose und Glucose enthaltenden Sirup zu erhalten.

7. Verfahren nach Anspruch 1, **dadurch gekennzeichnet, dass** die Isomaltulose und Trehalulose hydrolysierende α-Glucosidase, erhalten wird von Pilzen, vorzugsweise aus den Gattungen Aspergillus, Penicillum, Candida, Monilia, Phoma und Alternaria, bevorzugter von den Arten Aspergillus niger, Aspergillus oryzae oder Aspergillus awamori.

8. Verfahren nach Anspruch 1, **dadurch gekennzeichnet, dass** die Isomaltulose und Trehalulose hydrolysierende α-Glucosidase erhalten wird von Eubacteria, vorzugsweise von den Gattungen Enterobacter, Escherichia, Actinomyces, Bacillus, Klebsiella und Arthrobacter, bevorzugter von den Arten Bacillus subtilis, Bacillus licheniformis und Bacillus thermoamyloliquefaciens.

9. Verfahren nach Anspruch 1, **dadurch gekennzeichnet, dass** die Isomaltulose und Trehalulose hydrolysierende α-Glucosidase erhalten wird von Archaeobacteria, vorzugsweise der Gattungen Thermococcus, Thermoproteus, Pyrodictium und Halobacteria.

10. Verfahren nach Anspruch 1, **dadurch gekennzeichnet, dass** die Isomaltulose und Trehalulose hydrolysierende α-Glucosidase in immobilisierter Form, vorzugsweise immobilisiert auf einem Träger, bevorzugter immobilisiert auf einem wiederverwendbaren Träger, verwendet wird.

11. Verfahren nach Anspruch 10, **dadurch gekennzeichnet, dass** der Träger ein Anionenaustauschharz ist.

12. Verfahren nach Anspruch 11, **dadurch gekennzeichnet, dass** der Glucose und Fructose enthaltende Sirup mehr als 80 % Gew./Gew. Glucose und Fructose und weniger als 20 % Gew./Gew. Isomaltulose und Trehalulose umfasst und dass diese Werte mit einer Fliessgeschwindigkeit von bis zu 5 Bettvolumen pro Stunde und für einen Zeitraum von mindestens 60 Tagen erreicht werden.

13. Verfahren nach einem der Ansprüche 1 bis 12, wobei der Glucose und Fructose enthaltende Sirup weiter gereinigt wird, d.h. mit chromatographischen Mitteln oder durch Filtration behandelt wird.

14. Verwendung einer Isomaltulose und Trehalulose hydrolysierenden α-Glucosidase aus Eubacteria, Archaeobacteria oder Pilz zum Hydrolysieren eines Substrats umfassend ein Gemisch von Isomaltulose und Trehalulose umfassend mindestens 20 % Gew./Gew. Isomaltulose und Trehalulose, in einen Sirup, enthaltend mehr als 10 % Gew./Gew., vorzugsweise mehr als 25 % Gew./Gew., bevorzugter mehr als 40 % Gew./Gew., stärker bevorzugt mehr als 60 % Gew./Gew., am stärksten bevorzugt mehr als 80 % Gew./Gew. Glucose und Fructose.

## Revendications

1. Procédé pour hydrolyser de manière enzymatique un substrat **caractérisé en ce que** ledit procédé comprend les étapes suivantes :
a) prendre un substrat comportant un mélange d'isomaltulose et de tréhalulose comprenant au moins 20% en poids d'isomaltulose et de tréhalulose,
b) mettre ledit substrat en contact avec une alpha-glucosidase hydrolisant l'isomaltulose et le tréhalulose à partir d'eubactérie, d'archéobactérie ou de champignons ;
c) maintenir ledit contact pour hydrolyser le substrat pour obtenir un sirop contenant plus de 10% en poids, de préférence plus de 25% en poids, de manière davantage préférée plus de 40% en poids, de manière davantage préférée plus de 60% en poids, de manière préférée entre toutes plus de 80% en poids de glucose et de fructose,
d) éventuellement concentrer ledit sirop.

2. Procédé selon la revendication 1, **caractérisé en ce que**, entre l'étape c) et l'étape d) ou après l'étape d), le sirop est purifié de manière chromatographique en une fraction contenant du fructose et du glucose, et un courant secondaire contenant de l'isomaltulose et du tréhalulose, et ledit courant secondaire est recyclé dans l'étape b).

3. Procédé selon l'une quelconque des revendications 1 ou 2, **caractérisé en ce que** le mélange comprend entre 15% et 70% en poids d'isomaltulose et 5% à 30% en poids de tréhalulose.

4. Procédé selon l'une quelconque des revendications 1 à 3, **caractérisé en ce que** le mélange est la substance sèche de la liqueur mère obtenue à partir de la cristallisation de l'isomaltulose.

5. Procédé selon l'une quelconque des revendications 1 à 4, **caractérisé en ce que** le sirop contenant le glucose et le fructose comprend moins de 30% en poids d'isomaltulose et de tréhalulose, de préférence moins de 20% en poids d'isomaltulose et de tréhalulose, de manière davantage préférée moins de 10% en poids d'isomaltulose et de tréhalulose.

6. Procédé selon l'une quelconque des revendications 1 à 5, **caractérisé en ce que** le substrat est mis en contact avec l'alpha-glucosidase hydrolysant l'isomaltulose et le tréhalulose et le contact est maintenu à un pH en dessous de 6,5, de préférence en dessous de 6, de manière davantage préférée en dessous de 5 et à une température supérieure à 30°C, de préférence supérieure à 40°C, de manière davantage préférée supérieure à 50°C pour obtenir un sirop contenant du fructose et du glucose.

7. Procédé selon la revendication 1, **caractérisé en ce que** l'alpha-glucosidase hydrolysant l'isomaltulose et le trehalulose est obtenue à partir de champignons, de préférence à partir des genres Aspergillus, Penicillum, Candida, Monilia, Phoma, et Alternaria, de manière davantage préférée à partir des espèces *Aspergillus niger, Aspergillus oryzae ou Aspergillus awamori.*

8. Procédé selon la revendication 1, **caractérisé en ce que** l'alpha-glucosidase hydrolysant l'isomaltulose et le tréhalulose est une alpha-glucosidase obtenue à partir d'eubactéries, de préférence à partir des genres Enterobacter, Escherichia, Actinomyces, Bacillus, Klebsiella, et Arthrobacter, de manière davantage préférée à partir des espèces *Bacillus subtilis, Bacillus licheniformis,* et *Bacillus thermoamyloliquefaciens.*

9. Procédé selon la revendication 1, **caractérisé en ce que** l'alpha-glucosidase hydrolysant l'isomaltulose et le tréhalulose est une alpha-glucosidase obtenue à partir d'archéobactéries, de préférence à partir des genres Thermococcus, Thermoproteus, Pyrodictium et Halobacteria.

10. Procédé selon la revendication 1, **caractérisé en ce que** l'alpha-glucosidase hydrolysant l'isomaltulose et le tréhalulose est utilisée sous forme immobilisée, de préférence immobilisée sur un porteur, de manière davantage préférée immobilisée sur un porteur réutilisable.

11. Procédé selon la revendication 10, **caractérisé en ce que** le porteur est une résine échangeuse d'anion.

12. Procédé selon la revendication 11, **caractérisé en ce que** le sirop contenant du glucose et du fructose contient plus de 80% en poids de glucose et de fructose et moins de 20% en poids d'isomaltulose et tréhalulose, et que ces valeurs sont obtenues avec une vitesse d'écoulement jusqu'à 5 volumes de lit par heure et pour une période d'au moins 60 jours.

13. Procédé selon l'une quelconque des revendications 1 à 12, dans lequel le sirop contenant du glucose et du fructose est raffiné de manière supplémentaire c'est à dire traité par des moyens chromatographiques ou par filtration.

14. Utilisation d'une alpha-glucosidase hydrolysant l'isomaltulose et le tréhalulose à partir d'eubactéries, d'archéobactéries ou de champignons pour hydrolyser un substrat comprenant un mélange d'isomaltulose et de tréhalulose comprenant au moins 20% en poids d'isomaltulose et de trehalulose en un sirop contenant plus de 10% en poids, de préférence plus de 25% en poids, , de manière davantage préférée plus de 40% en poids, de manière davantage préférée plus de 60% en poids, de manière préférée entre toutes plus de 80% en poids de glucose et de fructose.
